# EUROPÄISCHE PATENTANMELDUNG

(11) **EP 2 072 143 A1**
(43) Veröffentlichungstag der Anmeldung: **24.06.2009**
(21) Anmeldenummer: 08386027.0
(22) Anmeldetag: 18.12.2008
(51) Int. Cl.: B05B 7/16, B05B 7/24, B05B 11/02, A61M 11/02, B05B 7/00, A01M 21/00, B01F 3/02

(54) **Zerstäuber**

(30) Priorität: 20.12.2007 GR 20070100765
(71) Anmelder: Kantzelis, Dimitrios, Ioannina 45 500 (GR); Fotikas, Vasilios, Ioannina (GR)
(72) Erfinder: Die Erfindernennung liegt noch nicht vor

(57) **Zusammenfassung**

Dieses Zerstäubungsgerät, das über eine tragbare Energiequelle betrieben wird, umfasst einen Flüssigkeitsbehälter (H), eine dosimetrische Spritze (E), eine Heizplatte (Δ), damit medikamentöse Substanzen zur Verdampfung eingebracht werden können, und umfasst auch einen Ventilator (θ) und eine dreieckige Einspritzdüse (A), damit die medikamentösen Substanzen versprüht werden können.
Außerdem kann man den Zerstäuber auch als elektrisches Räucherungsgerät (12 Volt) für Bienen, zum Spritzen von ultrakleinen Mengen (die Substanz mit überhaupt keinem oder geringstem Lösungsmittel) und als Musterentnahme von Gasen der Räume mit der Umpolung des Ventilators anwenden.

## Beschreibung

Der Zerstaeuber ist ein tragbares, leicht bedienbares und funktionelles Geraet.

Es wird mit einer unabhaengigen Energiequelle (12V) betrieben und dient sowohl als spezielles Geraet zur Bekaempfung von Zecken und anderen Bienenerkrankungen als auch als Spritzanlage kleinerer Mengen von Pestiziden, Medikamenten oder anderen Substanzen.

Das Geraet dient zudem ueber die Umpolung des Ventilators auch als Luft-Gas-Ansaugpumpe.

Die oben beschriebene Hauptnutzung des Geraets zur Bekaempfung von Zecken und anderen Bienenerkrankungen geschieht durch das Anbringen eines Zerstaeubungsschirmes oder Schlauchs an der Spitze der Einspritzduese, wodurch das Spritzen einfach und effizient wird. Das gewuenschte Resultat (Behandlung von Zecken und anderen Bienenerkrankungen) wird durch ein direktes Anbringen des Zerstaeubungsschirms oder der Zerstaeubungsduese am Eingang des Bienenstocks, ohne dessen Oeffnung, erreicht.

Eine wichtige Rolle fuer die funktionelle und substantive Nutzung des Geraets spielt der angeschlossene dosimetrische Einspritzmechanismus, dessen Spitze auf die Heizplatte in der Gaskammer muendet und ueber den die Medikamentenmenge exakt reguliert werden kann. Nach dem Verdampfen des Medikaments ist die Substanz mit der Betaetigung des Ventilators zur Zerstaeubung bereit.

Es ist vorgesehen und moeglich, auf der Heizplatte feste Substanzen mit Haken zu befestigen und diese mit Hilfe der Hitze gasfoermig zu machen.

Die vorgestellte Version des Zerstaeubungsgeraets hat eine durchsichtige Medikamentenkammer. In der Kammer ist am einen Ende ein Ventilator angebracht. Am anderen, gegenueberliegenden Ende der Kammer befindet sich die Einspritzduese zum Verspruehen der Medikamente.

Am Handgriff des Geraets sind der Ventilatorschalter und der Temperaturregler angebracht. Dadurch ist das Geraet leicht, funktionell und einfach zu bedienen. Auf dem Geraet sind eine Temperaturanzeige und eine Entnahmestelle zur Kontrolle des sich in der Kammer befindlichen Gasgemisches angebracht. Die Entnahmestelle ist mit einem Gummistopfen verschlossen.

Mit der Umpolung des Ventilators kann das Geraet auch zur Gasanalyse geschlossener Raeume verwendet werden. Hierbei wird die Raumluft in die Kammer angesaugt und ueber den Gummistopfen kann eine Probe zur Analyse des Gasgemisches entnommen werden.

Die im vorhergehenden beschriebene Funktion des Zerstaeubungsgeraetes machen es zu einem im Ganzen und im Detail effizienten Geraet, welches leicht zu bedienen und zu transportieren ist und mit einer einfachen Energiequelle (12V) betrieben wird.
- A.: Dreieckige Einspritzduese
- B.: Drehbarer Adaptor (Schlauch K)
- Γ.: Gaskammer
- Δ.: Heizplatte
- E.: Dosimetrische Spritze
- ΣT.: Ventilatorschalter (on/off)
- Z.: Heizplattenschalter (on/off)
- H.: Fluessigkeitsbehaelter
- Θ.: Ventilator
- I.: Seitenwaende der Heizplatte
- K.: Verbindungsschlauch zum Gasaustritt
- Λ.: Regulierbarer Thermostat
- M.: Gummistopfen
- N.: Ventilatorpolarisationsschalter
- Ξ.: Befestigunshaken fuer feste Substanzen
- O.: Stromkabel fuer 12V-Batterie
- II.: Temperaturanzeige fuer Heizplatte
- P.: Drehbarer Verbindungsring
- Σ.: Einspritzschlauch

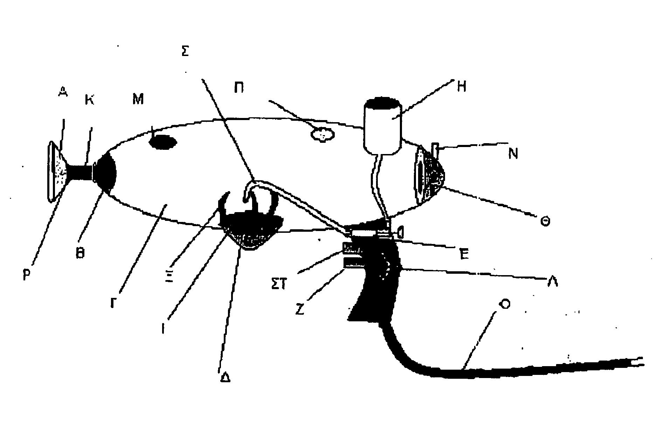

## Patentansprüche

1. Das Zerstaeubungsgeraet zeichnet sich **dadurch** aus, dass es ueber eine tragbare, unabhaengige Energiequelle betrieben wird. Medikamentoese Substanzen zur Bekaempfung von Zecken oder anderen Bienenerkrankungen koennen in fester oder fluessiger Form zur Verdampfung eingebracht und mit Hilfe des eingebauten Ventilators versprueht werden.
Dasselbe Geraet kann ausserdem ueber die Umpolung des Ventilators zur Entlueftung und Gasansaugung benutzt werden.

2. Nach dem Anspruch 1 ist das Zerstaeubungsgeraet durch eine Heizplatte, die sich in der durchsichtigen Gaskammer befindet, **gekennzeichnet**. An der Gaskammer ist ein Entlueftungsschlauch mit dosierbarer Einspritzduese zum Verspruehen der verdampften Medikamente angebracht.

3. Nach dem Anspruch 1 ist das Zerstaeubungsgeraet **dadurch gekennzeichnet, dass** der Fluessigkeitsbehaelter mit einer dosimetrisch regulierbaren Spritze verbunden ist, die sich am Handgriff befindet. Die Spritze endet mit einer Einspritzduese, die Medikamente in fluessiger Form zum Verdampfen auf die Heizplatte befoerdert und sie **dadurch** zum Zerstaeuben vorbereitet.

4. Nach dem Anspruch 1 ist das Zerstaeubungsgeraet **dadurch gekennzeichnet, dass** sich am Handgriff ein Schalter fuer die Heizplatte und ein Schalter fuer die Funktion des Ventilators befinden.
